# EUROPÄISCHE PATENTANMELDUNG

(11) **EP 4 516 245 A1**
(43) Veröffentlichungstag der Anmeldung: **05.03.2025**
(21) Anmeldenummer: 24195949.3
(22) Anmeldetag: 22.08.2024
(51) Int. Cl.: A61B 17/16, A61B 17/17, A61B 17/92

(54) **VORRICHTUNG ZUM PRÄPARIEREN EINES MARKRAUMS EINES KNOCHENS FÜR EIN IMPLANTAT, DIE VORRICHTUNG UMFASSENDES INSTRUMENTARIUM**

(30) Priorität: 28.08.2023 DE 102023123069
(71) Anmelder: Aesculap AG, 78532 Tuttlingen (DE)
(72) Erfinder: Nonnenmann, Martin, 78573 Wurmlingen (DE); Weindel, Sebastian, 78576 Emmingen-Liptingen (DE)
(74) Vertreter: DREISS Patentanwälte PartG mbB

(57) **Zusammenfassung**

Vorrichtung (100) zum Präparieren eines Markraums eines Knochens (210) für ein Implantat, wobei die Vorrichtung (100) einen Bohrer (104) zum Herstellen einer Bohrung im Markraum, eine Befestigungseinrichtung (106) für eine Raspel (108) zum Herstellen einer Öffnung im Markraum und ein Gehäuse (102) umfasst, wobei der Bohrer (104) relativ zum Gehäuse (102) translatorisch bewegbar angeordnet ist, wobei die Befestigungseinrichtung (106) relativ zum Gehäuse (102) translatorisch bewegbar angeordnet ist, wobei die Vorrichtung (100) eine Führung (112) umfasst, wobei die Führung (112) ausgebildet ist, den Bohrer (104) in einer translatorischen Bewegungsrichtung (110) des Bohrers (104) zum Herstellen der Bohrung und die Raspel (108) in der translatorischen Bewegungsrichtung oder in im Wesentlichen der translatorischen Bewegungsrichtung zum Herstellen der Öffnung zu führen. Die Vorrichtung umfassendes Instrumentarium.

## Beschreibung

Die Erfindung betrifft eine Vorrichtung zum Präparieren eines Markraums eines Knochens für ein Implantat und ein die Vorrichtung umfassendes Instrumentarium.

Zum Präparieren eines Markraums eines Knochens sind Bohrer zum Präparieren einer Bohrung zur Aufnahme eines rotationssymmetrischen Teils eines Implantats und Raspeln zum Präparieren von seitlichen der Bohrung angeordneten Öffnungen für seitlich desrotationssymmetrischen Teils angeordnete Verankerungen des Implantats in einem Markraum des Knochens bekannt.

Die Vorrichtung nach dem unabhängigen Anspruch 1 ermöglicht es die Bohrung und die seitlich dazu angeordneten Öffnungen mit einem Instrument zu präparieren.

Die Vorrichtung zum Präparieren eines Markraums eines Knochens für ein Implantat umfasst einen Bohrer zum Herstellen einer Bohrung im Markraum, eine Befestigungseinrichtung für eine Raspel zum Herstellen einer Öffnung im Markraum und ein Gehäuse, wobei der Bohrer relativ zum Gehäuse translatorisch bewegbar angeordnet ist, wobei die Befestigungseinrichtung relativ zum Gehäuse translatorisch bewegbar angeordnet ist, wobei die Vorrichtung eine Führung umfasst, wobei die Führung ausgebildet ist, den Bohrer in einer translatorischen Bewegungsrichtung des Bohrers zum Herstellen der Bohrung und die Raspel in der translatorischen Bewegungsrichtung oder in im Wesentlichen der translatorischen Bewegungsrichtung zum Herstellen der Öffnung zu führen.

Der Bohrer und die Raspel sind vorzugsweise in der translatorischen Bewegungsrichtung oder in im Wesentlichen der translatorischen Bewegungsrichtung relativ zueinander translatorisch beweglich angeordnet.

Die Führung umfasst vorzugsweise eine zumindest teilweise im Gehäuse angeordnete Führungseinrichtung, die ausgebildet ist, eine von der Führungseinrichtung geführte Bewegung des Bohrers in der translatorischen Bewegungsrichtung zuzulassen.

Die Führungseinrichtung ist vorzugsweise bezüglich des Gehäuses in der translatorischen Bewegungsrichtung oder in im Wesentlichen der translatorischen Bewegungsrichtung translatorisch bewegbar angeordnet.

Die Vorrichtung umfasst vorzugsweise eine Befestigungseinrichtung für die Raspel, wobei die Raspel an der Befestigungseinrichtung befestigt ist.

Die Raspel ist vorzugsweise an der Befestigungseinrichtung lösbar befestigt.

Die Führungseinrichtung weist vorzugsweise die Befestigungseinrichtung auf.

Die Führungseinrichtung ist vorzugsweise ausgebildet, Schlagenergie zur Bewegung der Führungseinrichtung in der translatorischen Bewegungsrichtung aufzunehmen und zur Bewegung der Befestigungseinrichtung in der translatorischen Bewegungsrichtung oder in im Wesentlichen der translatorischen Bewegungsrichtung an die Befestigungseinrichtung zu übertragen.

Die Führungseinrichtung ist vorzugsweise zumindest teilweise im Gehäuse angeordnet, wobei das Gehäuse einen Vorsprung aufweist, der als Anschlag für die Führungseinrichtung ausgebildet ist.

Die Befestigungseinrichtung ist vorzugsweise zumindest teilweise im Gehäuse angeordnet, wobei das Gehäuse einen Vorsprung aufweist, der als Anschlag für die Befestigungseinrichtung ausgebildet ist.

Die Führungseinrichtung weist vorzugsweise eine Einschlageinrichtung zur Aufnahme der Schlagenergie auf.

Das Gehäuse weist vorzugsweise eine Aussparung für die Einschlageinrichtung auf, in der der Einschlageinrichtung bezüglich des Gehäuses in der translatorischen Bewegungsrichtung bis zu einem als Anschlag ausgebildeten Ende der Aussparung bewegbar angeordnet ist.

Die Einschlageinrichtung weist vorzugsweise eine vom Gehäuse zumindest abschnittsweise radial zur translatorischen Bewegungsrichtung oder zu im Wesentlichen der translatorischen Bewegungsrichtung abstehende Fläche zur Aufnahme der Schlagenergie auf, wobei die Einschlageinrichtung an der Rückseite der Fläche einen Hinterschnitt aufweist.

Die Vorrichtung umfasst vorzugsweise eine Kupplungseinrichtung, die ausgebildet ist, zum Kuppeln des Bohrers und der Einschlageinrichtung bei einer Bewegung entgegen der translatorischen Bewegungsrichtung.

Die Führung umfasst vorzugsweise zumindest einen Teil einer Wand des Gehäuses, der ausgebildet ist, eine vom zumindest einen Teil der Wand geführte Bewegung der Raspel oder der Befestigungseinrichtung in der translatorischen Bewegungsrichtung oder in im Wesentlichen der translatorischen Bewegungsrichtung zuzulassen.

Die Vorrichtung umfasst vorzugsweise einen in oder einen entgegen der translatorischen Bewegungsrichtung wirkenden Anschlag für die translatorische Bewegung des Bohrers relativ zum Gehäuse.

Der Bohrer weist vorzugsweise einen Schaft auf, wobei zumindest ein Teil des Schafts in der Führungseinrichtung angeordnet ist, wobei der Teil des Schafts eine Verjüngung aufweist, wobei die Führungseinrichtung eine Verengung aufweist, die den Anschlag bildet.

Das Gehäuse umfasst vorzugsweise an seiner Außenseite einen Bereich, der als Handgriff ausgebildet ist.

Das Gehäuse umfasst vorzugsweise an der Außenseite eine Erhebung, die zwischen dem Bereich, der als Handgriff ausgebildet ist, und einem Bereich der Vorrichtung angeordnet ist, in dem ein Kopf des Bohrers oder die Raspel angeordnet ist.

Vorzugsweise ist im Bereich, der als Handgriff ausgebildet ist, wenigstens eine Kerbe angeordnet.

Die Raspel weist vorzugsweise wenigstens zwei Flügel auf, wobei zwischen den beiden Flügeln eine Aussparung für den Bohrer angeordnet ist.

Das Gehäuse weist vorzugsweise eine Ausschlageinrichtung zur Aufnahme von Schlagenergie entgegen der translatorischen Bewegungsrichtung oder entgegen im Wesentlichen der translatorischen Bewegungsrichtung auf, wobei die Ausschlageinrichtung eine vom Gehäuse zumindest abschnittsweise radial zur translatorischen Bewegungsrichtung oder zu im Wesentlichen der translatorischen Bewegungsrichtung abstehende Fläche zur Aufnahme der Schlagenergie aufweist.

Ein Instrumentarium zum Präparieren eines Markraums eines Knochens für ein Implantat, umfasst mindestens zwei auswechselbare Bohrer oder mindestens zwei auswechselbare Raspeln, wobei das Instrumentarium die Vorrichtung nach einem der vorherigen Ansprüche umfasst.

Das Instrumentarium umfasst vorzugsweise ein Präparationsplateau.

Weitere vorteilhafte Ausführungsformen sind der folgenden Beschreibung und der Zeichnung entnehmbar. In der Zeichnung zeigt:
- Fig. 1: eine schematische, perspektivische Darstellung von Teilen einer beispielhaften Ausführungsform einer Vorrichtung zum Präparieren eines Markraums eines Knochens für ein Implantat,
- Fig. 2: eine Explosionszeichnung von Teilen der beispielhaften Ausführungsform der Vorrichtung,
- Fig. 3: eine schematische, perspektivische Darstellung einer beispielhaften Flügelraspel,
- Fig. 4: eine schematische, perspektivische Darstellung eines beispielhaften Präparationsplateaus,
- Fig. 5: eine schematische, perspektivische Darstellung eines beispielhaften Adapters eines Motorensystems,
- Fig. 6: eine Seitenansicht auf die Vorrichtung,
- Fig. 7: eine Frontalsicht auf die Vorrichtung,
- Fig. 8: einen ersten Schnitt durch die Vorrichtung,
- Fig. 9: einen zweiten Schnitt durch die Vorrichtung,
- Fig. 10a bis 10d: eine schematische Darstellung einer Verwendung der Vorrichtung am Beispiel einer Präparation einer Tibia.

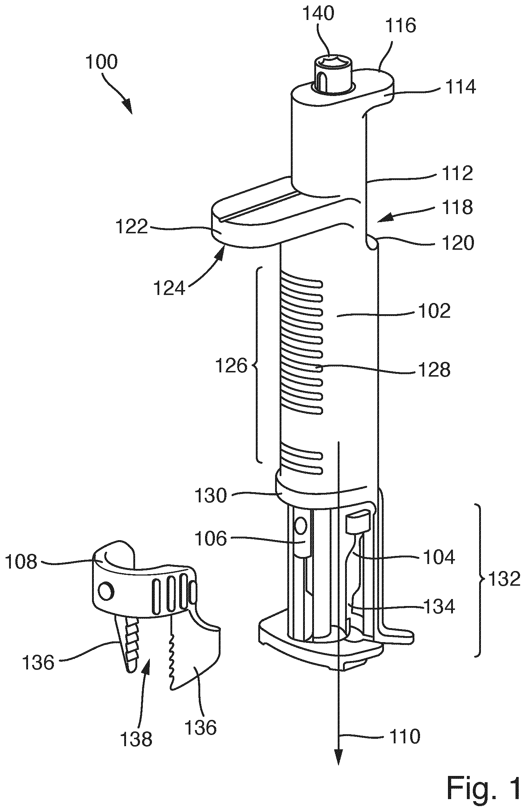

Figur 1 zeigt eine schematische, perspektivische Darstellung von Teilen einer Vorrichtung 100 zum Präparieren eines Markraums eines Knochens für ein Implantat. Die Vorrichtung 100 ist ein medizinisches Instrument.

Die Vorrichtung 100 zum Präparieren des Markraums wird am Beispiel eines Implantats für ein Knie, d.h. am Beispiel einer Knie-Endoprothese, beschrieben. Die Vorrichtung 100 ist im Beispiel zum Präparieren des Markraums einer Tibia ausgebildet.

Die Vorrichtung 100 umfasst ein Gehäuse 102, einen Bohrer 104 zum Herstellen einer Bohrung im Markraum und eine Befestigungseinrichtung 106 für eine Raspel 108 zum Herstellen einer Öffnung im Markraum.

Der Bohrer 104 ist relativ zum Gehäuse 102 translatorisch bewegbar angeordnet. Die Befestigungseinrichtung 106 ist relativ zum Gehäuse 102 translatorisch bewegbar angeordnet.

Der Bohrer 104 und die Raspel 108 sind in einer translatorischen Bewegungsrichtung 110 oder in im Wesentlichen der translatorischen Bewegungsrichtung relativ zueinander translatorisch beweglich angeordnet.

Die Vorrichtung 100 umfasst eine Führung. Die Führung ist ausgebildet, den Bohrer 104 in der translatorischen Bewegungsrichtung 110 des Bohrers 104 zum Herstellen der Bohrung zu führen.

Die Führung ist ausgebildet, die Raspel 108 in der translatorischen Bewegungsrichtung oder in im Wesentlichen der translatorischen Bewegungsrichtung zum Herstellen der Öffnung zu führen.

Die Führung umfasst im Beispiel zumindest einen Teil einer Wand des Gehäuses 102, der ausgebildet ist, eine vom zumindest einen Teil der Wand geführte Bewegung der Raspel 108 oder der Befestigungseinrichtung 106 in der translatorischen Bewegungsrichtung 110 oder in im Wesentlichen der translatorischen Bewegungsrichtung zuzulassen.

Die Führung umfasst beispielsweise eine zumindest teilweise im Gehäuse 102 angeordnete Führungseinrichtung 112. Die Führungseinrichtung 112 ist ausgebildet, eine von der Führungseinrichtung 112 geführte Bewegung des Bohrers 104 in der translatorischen Bewegungsrichtung 110 zuzulassen. Die Führungseinrichtung 112 ist ausgebildet, eine von der Führungseinrichtung 112 geführte Bewegung des Bohrers 104 entgegen der translatorischen Bewegungsrichtung 110 zuzulassen. Die Führungseinrichtung 112 ist ausgebildet, den Bohrer 104 in seiner Bohrachse in oder entgegen der translatorischen Bewegungsrichtung 110 zu führen. Die Führungseinrichtung 112 ist ausgebildet, eine von der Führungseinrichtung 112 geführte rotatorische Bewegung des Bohrers 104 um seine Bohrachse zuzulassen.

Die Führungseinrichtung 112 ist im Beispiel zumindest teilweise im Gehäuse 102 angeordnet.

Die Führungseinrichtung 112 ist bezüglich des Gehäuses 102 in der translatorischen Bewegungsrichtung 110 oder in im Wesentlichen der translatorischen Bewegungsrichtung translatorisch bewegbar angeordnet.

Die Raspel 108 ist an der Befestigungseinrichtung 106 befestigt. Die Raspel 108 ist im Beispiel an der Befestigungseinrichtung 106 lösbar befestigt. Beispielsweise sind Raspeln 108 unterschiedlicher Größe vorgesehen, die an der Befestigungseinrichtung 106 wahlweise lösbar befestigbar sind. Es kann vorgesehen sein, dass die Vorrichtung 100 mit einer an der Befestigungseinrichtung 106 unlösbar befestigten Raspel 108 ausgebildet ist.

Die Führungseinrichtung 112 ist ausgebildet, Schlagenergie zur Bewegung der Führungseinrichtung 112 in der translatorischen Bewegungsrichtung 110 aufzunehmen und zur Bewegung der Befestigungseinrichtung 112 in der translatorischen Bewegungsrichtung 110 oder in im Wesentlichen der translatorischen Bewegungsrichtung an die Befestigungseinrichtung 106 zu übertragen.

In einer beispielhaften Ausführungsform der Vorrichtung 100 weist die Führungseinrichtung 112 die Befestigungseinrichtung 106 auf.

In einer beispielhaften Ausführungsform der Vorrichtung 100 ist die Befestigungseinrichtung 106 als Stößel ausgebildet.

Die Befestigungseinrichtung 106 ist im Beispiel zumindest teilweise im Gehäuse 102 angeordnet.

Die Führungseinrichtung 112 weist eine Einschlageinrichtung 114 zur Aufnahme von Schlagenergie auf.

Die Einschlageinrichtung 114 umfasst im Beispiel eine vom Gehäuse 102 zumindest abschnittsweist radial zur translatorischen Bewegungsrichtung 110 oder zu im Wesentlichen der translatorischen Bewegungsrichtung abstehende Fläche 116 zur Aufnahme der Schlagenergie auf.

Das Gehäuse 102 weist im Beispiel eine Aussparung 118 für die Einschlageinrichtung 114 auf, in der der Einschlageinrichtung 114 bezüglich des Gehäuses 102 in der translatorischen Bewegungsrichtung 110 bewegbar angeordnet ist. Die Einschlageinrichtung 114 ist z.B. bis zu einem als Anschlag ausgebildeten Ende 120 der Aussparung bewegbar angeordnet.

Es kann vorgesehen sein, dass die Einschlageinrichtung 114 an der Rückseite der Fläche 116 einen Hinterschnitt aufweist, insbesondere einen Hinterschnitt, der mit einer Hand gut gegriffen werden kann. Durch den Hinterschnitt kann die Führungseinrichtung gut zurückgezogen werden.

Das Gehäuse 102 weist im Beispiel eine Ausschlageinrichtung 122 zur Aufnahme von Schlagenergie entgegen der translatorischen Bewegungsrichtung 110 oder entgegen im Wesentlichen der translatorischen Bewegungsrichtung auf.

Die Ausschlageinrichtung 122 umfasst z.B. eine vom Gehäuse 102 zumindest abschnittsweist radial zur translatorischen Bewegungsrichtung 110 oder zu im Wesentlichen der translatorischen Bewegungsrichtung abstehende Fläche 124 zur Aufnahme der Schlagenergie auf.

Das Gehäuse 102 umfasst im Beispiel an seiner Außenseite einen Bereich 126, der als Handgriff ausgebildet ist. Beispielsweise ist im Bereich 126, der als Handgriff ausgebildet ist, wenigstens eine Kerbe 128 angeordnet.

Das Gehäuse 102 umfasst im Beispiel an der Außenseite eine Erhebung 130, die zwischen dem Bereich 126, der als Handgriff ausgebildet ist, und einem Bereich 132 der Vorrichtung 100 angeordnet ist, in dem ein Kopf 134 des Bohrers 104 oder die Raspel 108 angeordnet ist.

Die wenigstens eine Kerbe 128 im Handgriff verbessern den Halt auch mit nassen, fettigen oder anderweitig mit Körperflüssigkeiten verschmutzten Handschuhen.

Durch die Erhebung 130 ist der Bereich 126 des Gehäuses 102 abgetrennt, der aufgrund von beweglichen Teilen nicht als Griffbereich geeignet ist.

Die Raspel 108 weist im Beispiel wenigstens zwei Flügel 136 auf. Es kann auch nur ein Flügel oder es können mehr als zwei Flügel, z.B. drei Flügel oder vier Flügel, vorgesehen sein.

Zwischen den Flügeln 136 ist eine Aussparung 138 für den Bohrer 104 angeordnet.

Der Bohrer 108 weist einen Schaft 140 auf. Zumindest ein Teil des Schafts 140 ist in der Führungseinrichtung 112 angeordnet.

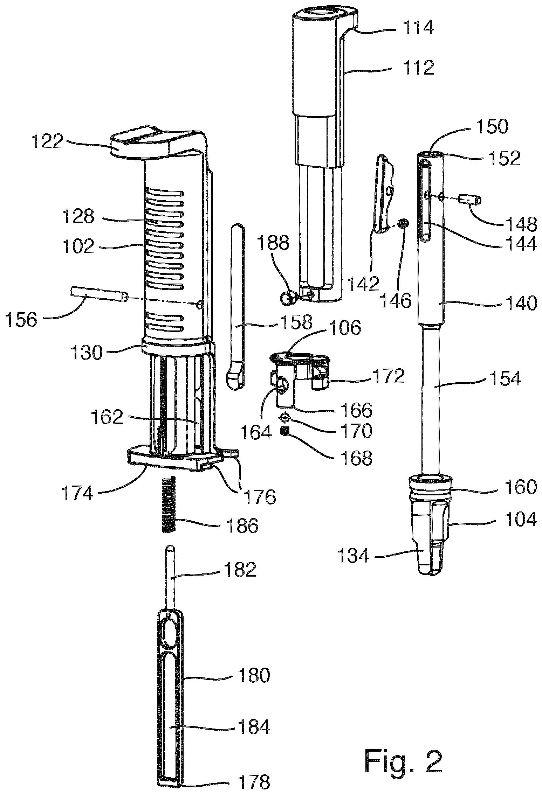

Figur 2 zeigt eine Explosionszeichnung von Teilen der beispielhaften Ausführungsform der Vorrichtung 100.

Die Vorrichtung 100 umfasst z.B. eine Kupplungseinrichtung, die ausgebildet ist, zum Kuppeln des Bohrers 104 und der Einschlageinrichtung 114 bei einer Bewegung entgegen der translatorischen Bewegungsrichtung 110.

Die Kupplungseinrichtung umfasst im Beispiel einen Riegel 142, der in einer Vertiefung 144 im Schaft 140 des Bohrers 104 angeordnet ist.

Der Bohrer 104 ist über den Riegel 142 mit der Einschlageinrichtung 114 verbunden. Der Bohrer 104 und die Einschlageinrichtung 114 sind durch den Riegel 142 derart verbunden, dass der Bohrer 104 und die Einschlageinrichtung 114 nach einer Präparation des Knochens gemeinsam in eine Ausgangsposition zurückstellbar sind. Der Riegel 142 ist z.B. als durch eine Feder 146 gefederte Wippe ausgeführt. Die Wippe umfasst im Beispiel einen Wippbalken, der von einem Stift 148 gebildet wird. Der Stift 148 wird im Beispiel in einander gegenüberliegenden Bohrungen in den Seitenwänden der Vertiefung 144 gehalten. Die Feder 146 ist im Beispiel eine Druckfeder, die sich an einer Rückwand der Vertiefung 144 abstützt.

Der Schaft 140 weist im Beispiel einen Adapter 150 für ein Motorensystem auf. Der Adapter 150 ist im Beispiel an einer Stirnseite des Bohrers 104 angeordnet. Der Adapter 150 weist eine Öffnung, z.B. einen Innensechskant, im Schaft 140 auf. Im Beispiel erstreckt sich die Öffnung von der Stirnseite bis zur Vertiefung 144.

Im Bereich des Adapters 150 weist der Schaft 140 zwischen dem Stift 148 und der Stirnseite ein Loch auf, das die Öffnung und die Vertiefung 114 verbindet. Ein Ende des Riegels 142 ist im Beispiel so positioniert, dass es durch die Öffnung nach außen bewegbar ist, wenn das Motorensystem in den Adapter 150 eingeführt wird. Dadurch öffnet sich der Riegel 142 automatisch, wenn das Motorensystem in die Öffnung im Schaft 140 des Bohrers 104 eingesetzt wird.

Alternativ dazu kann der Bohrer 104 eine Schnittstelle haben, welche eine direkte Kopplung mit einem Motorensystem ohne Adapter ermöglicht.

Es kann vorgesehen sein, dass das Motorensystem auch Bestandteil der Vorrichtung 100 ist.

Es kann vorgesehen sein, dass die Vorrichtung 100 einen in oder einen entgegen der translatorischen Bewegungsrichtung 110 wirkenden Anschlag für die translatorische Bewegung des Bohrers 104 relativ zum Gehäuse 102 umfasst. Beispielsweise weist der Teil des Schafts 140, der in der Führungseinrichtung 112 angeordnet ist, eine Verjüngung 154 auf. Beispielsweise weist die Führungseinrichtung 112 einen im Bereich der Verjüngung 154 durch zwei Bohrungen im Gehäuse 102 gehaltenen, quer zur Bewegungsrichtung 110 verlaufenden Stift 156 auf, der die beiden Anschläge für die nicht verjüngten Teile des Schafts 140 bildet.

Der Kopf 134 des Bohrers 104 weist im Beispiel eine Schneidengeometrie auf, die so ausgeführt ist, dass damit eine Bohrung für einen rotationssymmetrischen Teil des Implantats in der Tibia präpariert werden kann.

Der Bohrer 104 wird im Beispiel in einer Ausgangsposition im Gehäuse 102 durch eine in das Gehäuse 102 integrierte Feder 158 gehalten. Die Feder 158 ist z.B. als Blattfeder ausgebildet. Die Feder 158 greift im Beispiel in eine Nut 160, die am Umfang des Bohrers 104 angeordnet ist. Die Nut 160 ist z.B. zwischen dem Kopf 134 und der Verjüngung 154 angeordnet. Die Feder 158 und die Nut 160 halten den Bohrer 104 durch eine formschlüssige Verbindung bis zu einer definierten Kraft formschlüssig in der Ausgangsposition. Die formschlüssige Verbindung ist derart ausgeführt, dass sie sich ab einer definierten Kraft, die in Bohrrichtung auf den Bohrer 104 wirkt, automatisch entriegelt.

Es kann vorgesehen sein, dass das Gehäuse 102 wenigstens eine Öffnung 162 aufweist, die im Bereich 132 der Vorrichtung 100 angeordnet ist, in dem ein Kopf 134 des Bohrers 104 oder die Raspel 108 angeordnet ist. Durch die wenigstens eine Öffnung 162 wird das Gewicht des Gehäuses 102 reduziert und seine Reinigbarkeit verbessert.

Die Befestigungseinrichtung 106 umfasst im Beispiel eine quer zur translatorischen Bewegungsrichtung 110 verlaufende erste Bohrung 164 zur Aufnahme eines Zapfens der Raspel 108 auf. Im Beispiel ist in der ersten Bohrung 164 eine in translatorischer Bewegungsrichtung 110 verlaufende zweite Bohrung 166 angeordnet. In der zweiten Bohrung 166 ist sind eine Feder 168 und eine Kugel 170 angeordnet, die von der Feder 168 in die ersten Bohrung 164 gedrückt wird. Die Kugel 170 und die Feder 168 sind derart ausgebildet, dass die Kugel 170 in eine Nut am Zapfen der Flügelraspel einschnappt, wenn der Zapfen der Flügelraspel in die erste Bohrung 164 eingeführt wird.

Die Befestigungseinrichtung 106 umfasst einen quer zur translatorischen Bewegungsrichtung 110 angeordneten Bügel 172, der ausgebildet ist, den Bohrer 104 zu umgreifen. Der Bügel 172 weist im Beispiel zwei Enden auf, auf die jeweils eine quer zur translatorischen Bewegungsrichtung 110 angeordnete Nut an der Raspel 108 aufgeschoben werden kann.

Im Beispiel weist das Gehäuse 102 auf seiner in der translatorischen Bewegungsrichtung 110 dem Adapter 150 gegenüberliegenden Seite ein erster Teil einer Schnittstelle 174 zu einem Präparationsplateau auf. Der erste Teil der Schnittstelle 174 weist eine Öffnung für den Kopf 134 des Bohrers 104 und eine, die Öffnung umgebende Platte mit wenigstens einer Auskragung 176 in einem Randbereich der Platte auf.

Die Vorrichtung 100 umfasst eine insbesondere abgeschrägte Schnappnase 178. Die Schnappnase 178 ist an einem Schnappriegel 180 angeordnet, der in translatorischer Bewegungsrichtung 110 bewegbar im Gehäuse 102 angeordnet ist. Der Schnapprigel 180 umfasst auf seiner der Schnappnase 178 in der translatorischen Bewegungsrichtung 110 gegenüberliegenden Seite einen sich in translatorischer Bewegungsrichtung 110 erstreckenden ersten Stift 182. Zwischen dem Stift 182 und der Schnappnase 178 weist der Schnappriegel 180 ein sich in translatorischer Bewegungsrichtung 110 erstreckendes Langloch 184 auf. Der erste Stift 184 ist ausgebildet, eine Spiralfeder 186 zu führen, die auf den ersten Stift 184 aufgeschoben ist. Das Gehäuse 102 weist im Beispiel eine sich in translatorischer Bewegungsrichtung 110 erstreckende Hülse auf, in die der Stift 184 eintauchen kann. Die Spiralfeder 186 stützt sich an einem im Gehäuse 102 angeordneten Anschlag ab und drückt den Schnappriegel 180 aus bis zu einem Anschlag aus dem Gehäuse 102. Der Schnappriegel 180 ist über einen zweiten Stift 188, der sich quer zur translatorischen Bewegungsrichtung 110 aus der Führungseinrichtung 112 erstreckt, mit der Führungseinrichtung 112 gekoppelt.

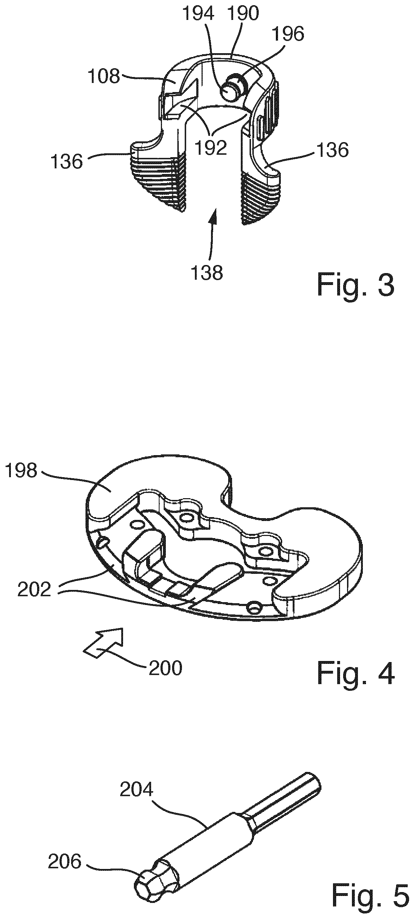

In Figur 3 ist eine schematische, perspektivische Darstellung einer beispielhaften Flügelraspel 108 dargestellt.

Die Flügelraspel 108 weist einen Bügel 190 auf, der ausgebildet ist, die Befestigungseinrichtung 106 zu umgreifen.

Der Bügel 190 weist im Beispiel beidseitig je eine Verbindungsnut 192 im Bügel 190 auf, die ausgebildet ist, auf den Bügel 176 der Befestigungseinrichtung 106, insbesondere auf deren Enden, aufgeschoben zu werden.

Die beiden Bügel sind ausgebildet, die Schlagenergie auf die Flügelraspel 108 zu übertragen.

Die Flügelraspel 108 umfasst einen Zapfen 194 der ausgebildet ist, in die erste Bohrung 164 der Befestigungseinrichtung 106 einzugreifen. Der Zapfen 194 umfasst eine radial um den Zapfen 194 umlaufende Nut 196, die ausgebildet ist, die Kugel 170 aufzunehmen. Die Kugel 170 schnappt in die Nut 196 am Zapfen 194 der Flügelraspel 108 ein, wenn der Zapfen 194 in die erste Bohrung 164 eingeschoben wird.

Die Bügel 176, 190, der Zapfen 194 und die Nut 192 sind Verbindungselemente, die quer zur Schlagrichtung verlaufen.

Das Einschnappen der Flügelraspel 108 mit diesen Verbindungselementen reicht als Verriegelung zwischen Flügelraspel 108 und Vorrichtung 100 aus und ermöglicht nach dem Einsatz der Vorrichtung 100 auch wieder ein einfaches Abziehen der Flügelraspel 108 ohne separate Bedienelemente betätigen zu müssen. Es kann vorgesehen sein, dass alle oder nur ein Teil der Verbindungselemente vorgesehen sind.

In Figur 4 ist eine schematische, perspektivische Darstellung eines beispielhaften Präparationsplateaus 198 dargestellt. Das Präparationsplateau 198 ist z.B. zur Präparation einer Tibia ausgebildet.

Im Beispiel sind die Vorrichtung 100 und das Präparationsplateau 198 dazu ausgebildet, dass die Vorrichtung 100 aus anteriorer Richtung 200 auf das Präparationsplateau 198 adaptiert wird.

Im Gegensatz zur Adaption aus proximaler Richtung führt die Adaption aus anteriorer Richtung zu weniger Konflikten mit dem Femur und den Weichteilen.

Das Präparationsplateau 198 umfasst einen zweiten Teil der Schnittstelle 202.

Die Schnittstelle ist im Beispiel als winklig zulaufende Schwalbenschwanzführung ausgelegt.

Durch die beiden Winkel hat die Schnittstelle zu Beginn der Kopplungsbewegung viel Spiel und kann so auch bei einem leichten Versatz oder Winkelfehler zwischen den beiden Bauteilen gefügt werden. Beim Verbinden zentriert sich die Vorrichtung 100 somit automatisch auf dem Präparationsplateau 198.

Die Vorrichtung 100 und das Präparationsplateau 198 können alternativ mit auch einer Schnittstelle zum ausgeführt werden, die in proximale Richtung gekoppelt und entkoppelt werden kann.

Die Vorrichtung 100 kann alternativ dazu eine Auflagefläche umfassen, die so gestaltet ist, dass sie ohne Präparationsplateau 198 direkt auf dem Knochen aufgesetzt werden kann. In die Auflagefläche integrierte raue Strukturen oder Spikes können dabei optional ein mögliches Verrutschen verhindern. Es können optionale Bohrungen in der Vorrichtung 100 vorgesehen sein, die ausgebildet sind, eine Fixierung der Vorrichtung 100 direkt auf dem Knochen mittels Schraub- oder Schlagpins zu ermöglichen.

Wird die Vorrichtung 100 bis zum Anschlag gekoppelt, verriegelt sie sich automatisch über die insbesondere abgeschrägte Schnappnase 178.

In Figur 5 eine schematische, perspektivische Darstellung eines beispielhaften Adapters 204 des Motorensystems dargestellt.

Der Adapter 204 des Motorensystems umfasst z.B. einen Außensechskantadapter 206. Ein Sechskantende 206 des Adapters 204 ist im Beispiel kugelabschnittsförmig gestaltet. Dadurch kann der Adapter 204 auch Rotationsbewegungen übertragen, wenn die Winkel der Achsen des Bohrers und des Motorensystems nicht exakt übereinstimmen.

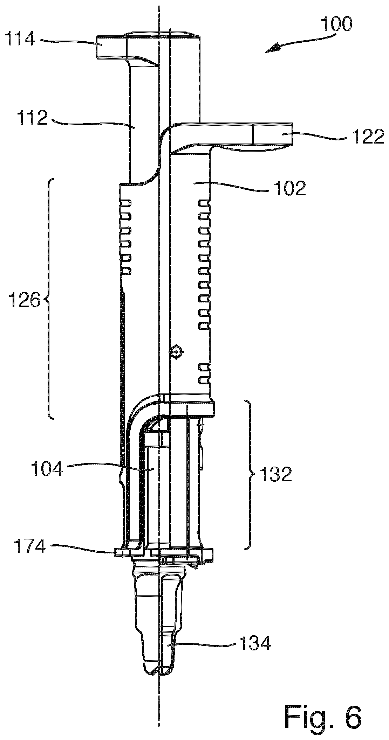

In Figur 6 ist eine Seitenansicht auf die Vorrichtung 100 dargestellt. In der Seitenansicht ist der Kopf 134 des Bohrers 104 aus dem Gehäuse 102 durch die Schnittstelle 174 ausgeschoben. Dies stellt eine Arbeits-Endposition des Bohrers 104 dar.

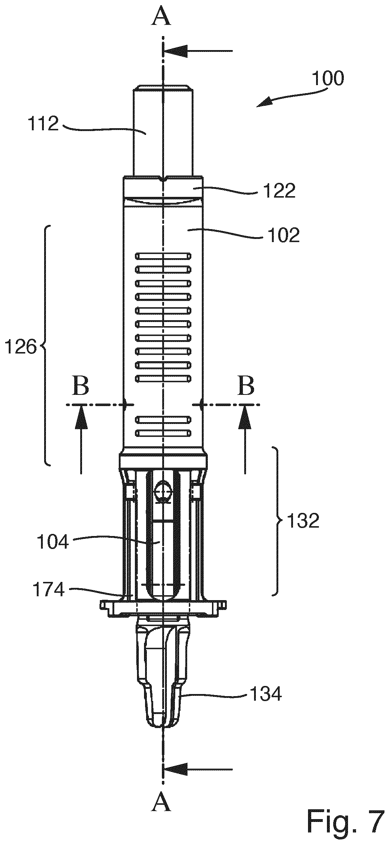

In Figur 7 ist eine Frontalsicht auf die Vorrichtung 100 dargestellt. In der Frontalsicht ist der Bohrers 104 in der Arbeits-Endposition.

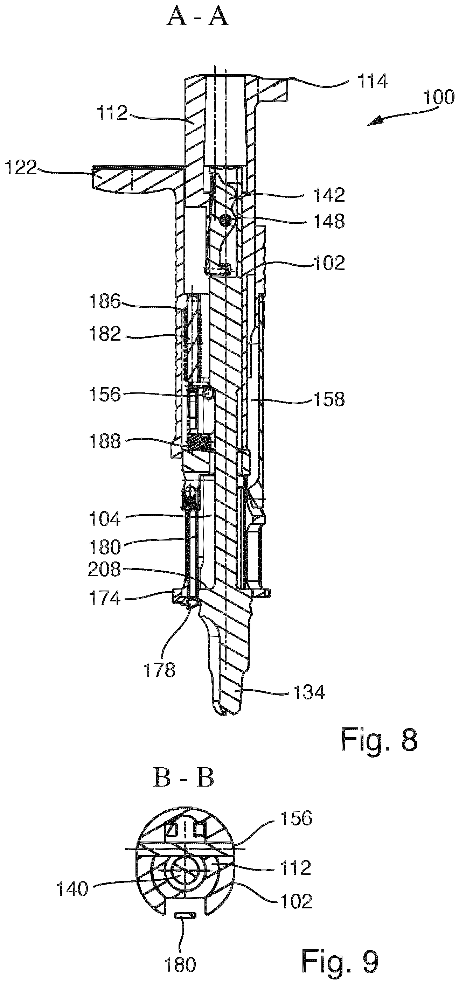

In Figur 8 ist ein erster Schnitt A-A durch die Vorrichtung 100 dargestellt, in der der Bohrer 104 in der Arbeits-Endposition angeordnet ist.

Das Gehäuse 102 weist einen Vorsprung 208 auf.

Der Vorsprung 108 ist als Tiefenanschlag für die Führungseinrichtung 112 ausgebildet oder für die Befestigungseinrichtung 106 ausgebildet.

Da in der jeweiligen Arbeits-Endposition des Bohrers 104 und der Führungseinrichtung 112 der Riegel 142 des Bohrers in die Führungseinrichtung 112 einrastet, wird durch das Zurückziehen der Führungseinrichtung 112 gleichzeitig auch der Bohrer 104 in die Ausgangsposition zurückgezogen. Gleichzeitig zieht der Stift 188 an der Führungseinrichtung 112 den Schnappriegel 180 mit zurück und entriegelt somit des Präparationsplateau 198 von der Vorrichtung 100.

In Figur 9 ist einen zweiten Schnitt B-B durch die Vorrichtung 100 im Bereich des Stifts 156, der die beiden Anschläge für die nicht verjüngten Teile des Schafts 140 des Bohrers 104 bildet dargestellt.

Die Anschläge sind derart ausgeführt, dass das eine für das jeweilige Implantat passende Präparationstiefe gewährleistet ist.

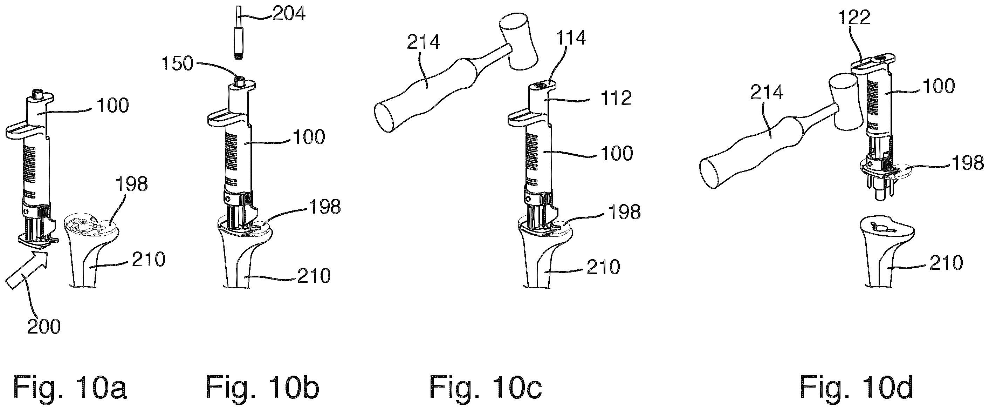

In Figuren 10a bis 10d ist eine Verwendung der Vorrichtung 100 am Beispiel einer Präparation einer Tibia 210 schematisch dargestellt.

Figur 10a zeigt die Tibia 210, auf der bereits das Präparationsplateau 198 angeordnet ist. Die Vorrichtung 100 wird aus anteriorer Richtung 200 auf das Präparationsplateau 198 adaptiert.

Figur 10b zeigt die auf Präparationsplateau 198 angeordnete Vorrichtung 100. Im Beispiel ist die Schnappnase 178 des Schnappriegels 180 im Präparationsplateau 198 eingerastet. Mit dem Bohrer 104 wird das Bohrloch in der Tibia 210 präpariert. Das Motorsystem dreht den Bohrer 104 über den Adapter 204 des Motorsystem und den Adapter 150 der Vorrichtung 100.

Fig. 10c zeigt das Einschlagen der Raspel 108 mit einem Hammer 214. Die Einschlagplatte 114 am oberen Ende der Vorrichtung 100 überträgt über die Führungseinrichtung 112 die Schlagenergie des Hammers 214 auf die Befestigungseinrichtung 106 für die Raspel 108. Dadurch wird die mit der Befestigungseinrichtung 106 verbundene Raspel 108 in die Tibia 210 eingeschlagen.

Da während des Einschlagvorgangs der Raspel 108 auch noch zentral der Bohrer 104 in der Tibia 210 sitzt, wird eine Winkelstabilität beim Einschlagen erreicht, die die Stabilität von bisher üblichen getrennten Bohr- und Raspel-Instrumenten übertrifft.

Fig. 10d zeigt das Ausschlagen. Die fest mit dem Gehäuse 102 der Vorrichtung 100 verbundene Ausschlagplatte 122 ermöglicht, die komplette Vorrichtung 100, einschließlich Bohrer 104 und Raspel 108 auszuschlagen.

Die Vorrichtung 100 ist zudem mittels des Schnappriegels 180 im Präparationsplateau 198 eingerastet. Somit können in einem Arbeitsschritt der Bohrer 104, die Raspel 108, und das mit der Vorrichtung 100 verbundene Präparationsplateau 198 entfernt werden.

Das Präparationsplateau 198 kann Verankerungspins zur Verankerung des Präparationsplateau 198 in der Tibia 210 umfassen. Die Verankerungspins werden in diesem Fall zusammen mit dem Präparationsplateau 198 entfernt.

Es kann vorgesehen sein, dass die Auskragungen 176 derart am Gehäuse 102 angeordnet sind, dass sie die Verankerungspins im Präparationsplateau 198 überdecken. Beim Ausschlagen der Vorrichtung 100 zusammen mit dem Präparationsplateau 198 können die Verankerungspins dann nicht versehentlich herausfallen.

Die Vorrichtung 100 bzw. die Vorrichtung 100 und das Präparationsplateau 198 kann so als Einheit zusammen mit dem Bohrer 104, der Raspel 108, dem Präparationsplateau 198 und ggf. den Verankerungspins vom Operateur an die assistierende Person übergeben werden.

Für ein Implantatsystem, das unterschiedliche tiefe Bohrungen erfordert, können unterschiedliche lange Bohrer 104 vorgesehen sein, wobei je einer der Bohrer 104 demontierbar im Gehäuse 102 angeordnet ist. Alternativ dazu kann für einen nicht demontierbaren Bohrer 104 einen verstellbaren Tiefenanschlag im Gehäuse angeordnet sein. Es können Bohrer 104 mit derselben rotationssymetrischen Geometrie oder mit unterschiedlichen Geometrien vorgesehen sein.

Dadurch kann eine Geometrie für einen rotationssymmetrischen Zapfen zur Verankerung im Knochen mit unterschiedliche Langen Bohrern präpariert werde.

Es kann vorgesehen sein, dass die Vorrichtung 100 eine oder mehrere Schnittstellen für einen Schlaghammer mit einem Schlaggewicht aufweist. Dadurch kann zum Ein- und/oder Ausschlagen ein Schlaghammer mit geführtem Schlaggewicht verwendet werden.

Für ein Implantatsystem, das unterschiedliche tiefe mit der Raspel 108 zu präparierende Öffnungen erfordert, können unterschiedlich tiefe Raspeln 108 oder ein verstellbarer Tiefenanschlag für die Raspel 108 vorgesehen sein.

Für ein Implantatsystem, das unterschiedliche Anordnungen der Flügel der Raspel 108 erfordert, die unterschiedlich zueinander oder zur zentralen Bohrung positioniert sind, können die Flügel 136 der Raspel 108 demontierbar oder nicht demontierbar an der Vorrichtung 100 verbaubar ausgeführt und/oder in ihre Position an der Vorrichtung 100 verstellbar sein.

Ein Instrumentarium zum Präparieren des Markraums umfasst z.B. die Vorrichtung 100, wobei mindestens zwei auswechselbare Raspeln 108 unterschiedlicher Länge, Breite oder unterschiedlicher Anordnung der Flügel 136 vorgesehen sind.

Es kann vorgesehen sein, dass die Vorrichtung 100 für auswechselbare Bohrer ausgebildet ist, wobei das Instrumentarium mindestens zwei auswechselbare Bohrer 104 unterschiedlicher Länge oder mit unterschiedlichem Durchmesser umfasst.

Es kann vorgesehen sein, dass das Instrumentarium das Präparationsplateau 198 umfasst.

## Patentansprüche

1. Vorrichtung (100) zum Präparieren eines Markraums eines Knochens (210) für ein Implantat, **dadurch gekennzeichnet, dass** die Vorrichtung (100) einen Bohrer (104) zum Herstellen einer Bohrung im Markraum, eine Befestigungseinrichtung (106) für eine Raspel (108) zum Herstellen einer Öffnung im Markraum und ein Gehäuse (102) umfasst, wobei der Bohrer (104) relativ zum Gehäuse (102) translatorisch bewegbar angeordnet ist, wobei die Befestigungseinrichtung (106) relativ zum Gehäuse (102) translatorisch bewegbar angeordnet ist, wobei die Vorrichtung (100) eine Führung (112) umfasst, wobei die Führung (112) ausgebildet ist, den Bohrer (104) in einer translatorischen Bewegungsrichtung (110) des Bohrers (104) zum Herstellen der Bohrung und die Raspel (108) in der translatorischen Bewegungsrichtung oder in im Wesentlichen der translatorischen Bewegungsrichtung zum Herstellen der Öffnung zu führen.

2. Vorrichtung nach Anspruch 1, **dadurch gekennzeichnet, dass** der Bohrer (104) und die Raspel (108) in der translatorischen Bewegungsrichtung (110) oder in im Wesentlichen der translatorischen Bewegungsrichtung relativ zueinander translatorisch beweglich angeordnet sind.

3. Vorrichtung nach einem der vorherigen Ansprüche, **dadurch gekennzeichnet**, die Führung (110, 112) eine zumindest teilweise im Gehäuse (102) angeordnete Führungseinrichtung (112) umfasst, die ausgebildet ist, eine von der Führungseinrichtung (112) geführte Bewegung des Bohrers (104) in der translatorischen Bewegungsrichtung (110) zuzulassen.

4. Vorrichtung (100) nach Anspruch 3, **dadurch gekennzeichnet, dass** die Führungseinrichtung (112) bezüglich des Gehäuses (102) in der translatorischen Bewegungsrichtung (110) oder in im Wesentlichen der translatorischen Bewegungsrichtung translatorisch bewegbar angeordnet ist.

5. Vorrichtung (100) nach Anspruch 3 oder 4, **dadurch gekennzeichnet, dass** die Vorrichtung (100) eine Befestigungseinrichtung (112) für die Raspel (108) umfasst, wobei die Raspel (108) an der Befestigungseinrichtung (112) befestigt ist.

6. Vorrichtung (100) nach Anspruch 5, **dadurch gekennzeichnet, dass** die Raspel (108) an der Befestigungseinrichtung (106) lösbar befestigt ist.

7. Vorrichtung (100) nach Anspruch 5 oder 6, **dadurch gekennzeichnet, dass** die Führungseinrichtung (112) die Befestigungseinrichtung (106) aufweist.

8. Vorrichtung (100) nach Anspruch 5 oder 6, **dadurch gekennzeichnet, dass** die Führungseinrichtung (112) ausgebildet ist, Schlagenergie zur Bewegung der Führungseinrichtung (112) in der translatorischen Bewegungsrichtung (110) aufzunehmen und zur Bewegung der Befestigungseinrichtung (106) in der translatorischen Bewegungsrichtung (110) oder in im Wesentlichen der translatorischen Bewegungsrichtung (110) an die Befestigungseinrichtung (108) zu übertragen.

9. Vorrichtung (100) nach Anspruch 8, **dadurch gekennzeichnet, dass** die Führungseinrichtung (112) zumindest teilweise im Gehäuse (102) angeordnet ist, wobei das Gehäuse (102) einen Vorsprung aufweist, der als Anschlag für die Führungseinrichtung (112) ausgebildet ist.

10. Vorrichtung (100) nach Anspruch 8 oder 9, **dadurch gekennzeichnet, dass** die Befestigungseinrichtung (106) zumindest teilweise im Gehäuse (102) angeordnet ist, wobei das Gehäuse einen Vorsprung aufweist, der als Anschlag für die Befestigungseinrichtung (106) ausgebildet ist.

11. Vorrichtung (100) nach einem der Ansprüche 8 bis 10, **dadurch gekennzeichnet, dass** die Führungseinrichtung (112) eine Einschlageinrichtung (114) zur Aufnahme der Schlagenergie aufweist.

12. Vorrichtung (100) nach Anspruch 11, **dadurch gekennzeichnet, dass**, wobei das Gehäuse (102) eine Aussparung (118) für die Einschlageinrichtung (114) aufweist, in der der Einschlageinrichtung (114) bezüglich des Gehäuses (102) in der translatorischen Bewegungsrichtung (110) bis zu einem als Anschlag ausgebildeten Ende der Aussparung (118) bewegbar angeordnet ist.

13. Vorrichtung (100) nach einem der Ansprüche 11 oder 12, **dadurch gekennzeichnet, dass** die Einschlageinrichtung (114) eine vom Gehäuse (102) zumindest abschnittsweise radial zur translatorischen Bewegungsrichtung (110) oder zu im Wesentlichen der translatorischen Bewegungsrichtung (110) abstehende Fläche (116) zur Aufnahme der Schlagenergie aufweist, wobei die Einschlageinrichtung (114) an der Rückseite der Fläche (116) einen Hinterschnitt aufweist.

14. Vorrichtung (100) nach einem der Ansprüche 11 bis 13, **dadurch gekennzeichnet, dass** die Vorrichtung (100) eine Kupplungseinrichtung umfasst, die ausgebildet ist, zum Kuppeln des Bohrers (104) und der Einschlageinrichtung (114) bei einer Bewegung entgegen der translatorischen Bewegungsrichtung (110).

15. Vorrichtung (100) nach einem der vorherigen Ansprüche, **dadurch gekennzeichnet, dass** die Führung (112) zumindest einen Teil einer Wand des Gehäuses (102) umfasst, der ausgebildet ist, eine vom zumindest einen Teil der Wand geführte Bewegung der Raspel (108) oder der Befestigungseinrichtung (106) in der translatorischen Bewegungsrichtung (110) oder in im Wesentlichen der translatorischen Bewegungsrichtung zuzulassen.

16. Vorrichtung (100) nach einem der vorherigen Ansprüche, **dadurch gekennzeichnet, dass** die Vorrichtung (100) einen in oder einen entgegen der translatorischen Bewegungsrichtung (110) wirkenden Anschlag für die translatorische Bewegung des Bohrers (104) relativ zum Gehäuse (102) umfasst.

17. Vorrichtung (100) nach Anspruch 10, **dadurch gekennzeichnet, dass** der Bohrer (108) einen Schaft (140) aufweist, wobei zumindest ein Teil des Schafts (140) in der Führungseinrichtung (112) angeordnet ist, wobei der Teil des Schafts (140) eine Verjüngung aufweist, wobei die Führungseinrichtung (112) eine Verengung aufweist, die den Anschlag bildet.

18. Vorrichtung nach einem der vorherigen Ansprüche, **dadurch gekennzeichnet, dass** das Gehäuse (102) an seiner Außenseite einen Bereich (126) umfasst, der als Handgriff ausgebildet ist.

19. Vorrichtung (100) nach Anspruch 18, **dadurch gekennzeichnet, dass** das Gehäuse (102) an der Außenseite eine Erhebung (130) umfasst, die zwischen dem Bereich (126), der als Handgriff ausgebildet ist, und einem Bereich (132) der Vorrichtung (100) angeordnet ist, in dem ein Kopf (134) des Bohrers (104) oder die Raspel (108) angeordnet ist.

20. Vorrichtung (100) nach Anspruch 18 oder 19, **dadurch gekennzeichnet, dass** im Bereich (126), der als Handgriff ausgebildet ist, wenigstens eine Kerbe (128) angeordnet ist.

21. Vorrichtung (100) nach einem der vorherigen Ansprüche, **dadurch gekennzeichnet, dass** die Raspel (108) wenigstens zwei Flügel (136) aufweist, wobei zwischen den beiden Flügeln (136) eine Aussparung (138) für den Bohrer (104) angeordnet ist.

22. Vorrichtung (100) nach einem der vorherigen Ansprüche, **dadurch gekennzeichnet, dass** das Gehäuse (102) eine Ausschlageinrichtung (122) zur Aufnahme von Schlagenergie entgegen der translatorischen Bewegungsrichtung (110) oder entgegen im Wesentlichen der translatorischen Bewegungsrichtung aufweist, wobei die Ausschlageinrichtung (122) eine vom Gehäuse (102) zumindest abschnittsweise radial zur translatorischen Bewegungsrichtung (110) oder zu im Wesentlichen der translatorischen Bewegungsrichtung abstehende Fläche (124) zur Aufnahme der Schlagenergie aufweist.

23. Instrumentarium zum Präparieren eines Markraums eines Knochens (210) für ein Implantat, umfassend mindestens zwei auswechselbare Bohrer (104) oder mindestens zwei auswechselbare Raspeln (108), **dadurch gekennzeichnet, dass** das Instrumentarium die Vorrichtung (100) nach einem der vorherigen Ansprüche umfasst.

24. Instrumentarium nach Anspruch 23, **dadurch gekennzeichnet, dass** das Instrumentarium ein Präparationsplateau (198) umfasst.
